# EUROPEAN PATENT SPECIFICATION

(11) **EP 2 740 510 B1**
(45) Date of publication and mention of the grant of the patent: **01.11.2017**
(21) Application number: 12820051.6
(22) Date of filing: 30.07.2012
(51) Int. Cl.: A61M 29/00, A61B 17/34

(54) **DILATOR**
DILATATOR
DILATATEUR

(30) Priority: 01.08.2011 JP 2011168776
(43) Date of publication of application: 11.06.2014
(73) Proprietor: Terumo Kabushiki Kaisha, Tokyo 151-0072 (JP)
(72) Inventor: OKAMURA, Ryo, Fujinomiya-shi Shizuoka 418-0015 (JP); SHINOHARA, Toshiaki, Fujinomiya-shi Shizuoka 418-0015 (JP)
(74) Representative: Casalonga
(86) International application number: PCT/JP2012/069366
(87) International publication number: WO 2013/018771

(56) References cited:
- WO-A2-2007/046850
- JP-A- 2002 191 697
- JP-U- 3 053 402
- US-A1- 2003 083 688

## Description

### Technical Field

The present invention relates to a dilator which is used for diameter expansion of an introduction hole communicating with the inside of a living body.

### Background Art

A Seldinger method has been known in the related art, as a method of percutaneously introducing a catheter into a living body such as a blood vessel. In the Seldinger method, after forming an introduction hole which communicates with the inside of a living body by an introduction needle which punctures the skin, an elongated dilator is inserted into the introduction hole in a state where the dilator is inserted through the inside of a tubular introducer sheath through which a catheter can be inserted.

The dilator protrudes from a distal end of the introducer sheath, and expands a diameter of the introduction hole with a taper-shaped distal portion while passing through the introduction hole. Accordingly, an operator senses resistance at the time of inserting the dilator into the introduction hole, and when the operator strongly inserts the dilator to resist such resistance, physical strain is imposed to a patient. Here, a proposal is made to reduce the resistance.

In a dilator disclosed in PTL 1, for example, by providing a groove or a protrusion which extends in an axial direction on the surface of a tapered-shaped distal portion, decrease in contact resistance between a blood vessel and the dilator or between a tissue and the dilator is realized.

### Citation List

### Patent Literature

PTL 1: Japanese Registered Utility Model No. 3053402

### Summary of Invention

### Technical Problem

However, when the inventors examined the decrease in resistance of the dilator using the conventional technology, the inventors practically still sensed great resistance. The reason for this is considered that, when a largest outer diameter portion of a distal portion of a dilator passes through an introduction hole, rapid change in resistance generated before and after the passage occurs. That is, although a slit-shaped groove or protrusion having a greater depth than a width is provided on the surface of the distal portion of the dilator as the related art, the rapid change in the resistance generated when the largest outer diameter portion passes through the introduction hole is not suppressed, and as a result an operator senses great resistance.

The present invention has been made to address the aforementioned problems and an object thereof is to provide a dilator which can suppress rapid change in resistance and can be smoothly inserted into an introduction hole.

### Solution to Problem

As a result of performing investigation to solve the problems, the inventors have found that rapid change in resistance generated when a dilator passes through an introduction hole is suppressed, by providing a cross-sectional area reduction portion having a predetermined shape with a reduced part of an outer periphery of a circular cross section, on a largest outer diameter portion of a distal portion of the dilator or a portion in which a rate of increase of an outer diameter in the distal portion of the dilator changes, and the present invention is completed.

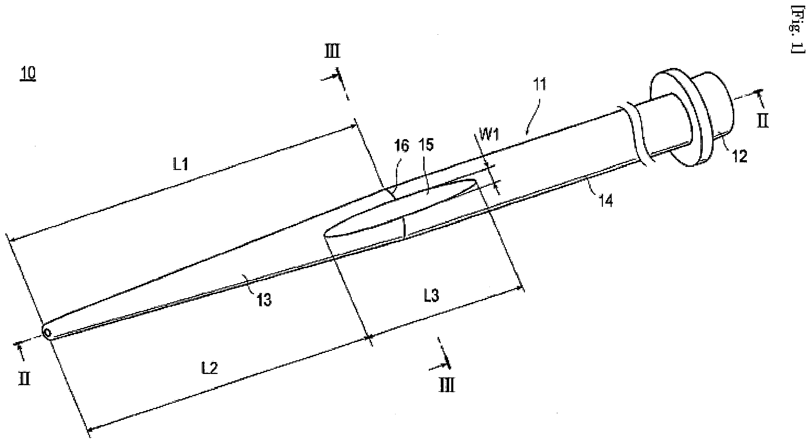

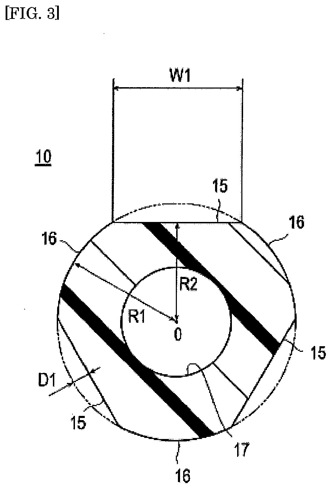

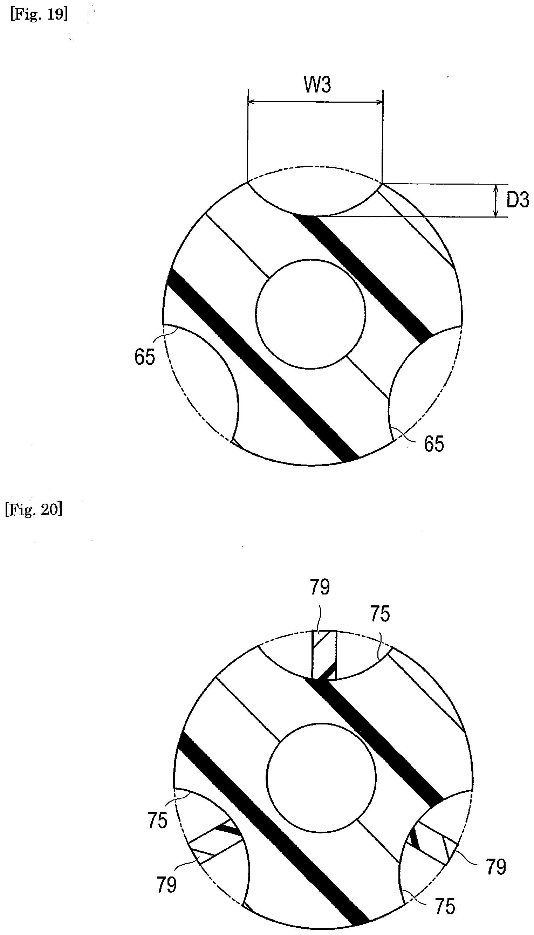

That is, there is provided a dilator for achieving the object, including: a distal portion, an outer diameter of which increases towards a proximal end side in an axial direction; and a cross-sectional area reduction portion which is provided at least on a largest outer diameter portion of the distal portion, and is obtained by reducing a part of an outer periphery of a circular cross section, in which a width of the cross-sectional area reduction portion in a chord direction is equal to or greater than a depth of the cross-sectional area reduction portion in a direction perpendicular to the chord direction. The width of the cross-sectional area reduction portion in the chord direction indicates a length of a straight-line distance between intersection points of an outer periphery of a circular cross section and the cross-sectional area reduction portion, in the circular cross section of the largest outer diameter portion of the distal portion of the dilator. That is, the width of the cross-sectional area reduction portion in the chord direction indicates a width of the cross-sectional area reduction portion in a circumferential direction of the largest outer diameter portion of the distal portion of the dilator, and is a length of W1 in Fig. 1. In addition, the depth of the cross-sectional area reduction portion in a direction perpendicular to the chord direction of the cross-sectional area reduction portion is a difference between a length from the center of a circular cross section to an outer periphery on a circular arc which is not a cross-sectional area reduction portion, and a length of a perpendicular line from the center of the circular cross section to the cross-sectional area reduction portion, in the circular cross section of the largest outer diameter portion of the distal portion of the dilator. That is, the depth of the cross-sectional area reduction portion in a direction perpendicular to the chord direction of the cross-sectional area reduction portion indicates a length from the outer periphery of the circular cross section which is not a cross-sectional area reduction portion to a flat surface of the cross-sectional area reduction portion, when drawing a perpendicular line from the outer periphery of the circular cross section which is not a cross-sectional area reduction portion towards the center of the circular cross section, in the cross-sectional area reduction portion of the largest outer diameter portion of the distal portion of the dilator, and is a length of D1 in Fig. 3. In a case where the cross-sectional area reduction portion is formed on a recessed curved surface as shown in Fig. 19, the depth of the cross-sectional area reduction portion in a direction perpendicular to the chord direction of the cross-sectional area reduction portion is a difference between a length of a perpendicular line from a straight line connecting intersection points of an outer periphery of a circular cross section and the cross-sectional area reduction portion to the center of the circular cross section, and a length of a perpendicular line from the cross-sectional area reduction portion to the center of the circular cross section, and is a length of D3 in Fig. 19.

In addition, there is provided a dilator of the present invention for achieving the obj ect, including: a distal portion, an outer diameter of which increases towards a proximal end side in an axial direction; and a cross-sectional area reduction portion which is provided at least on a portion in which a rate of increase of the outer diameter of the distal portion changes, and is obtained by reducing a part of an outer periphery of a circular cross section, in which a width of the cross-sectional area reduction portion in a chord direction is equal to or greater than a depth of the cross-sectional area reduction portion in a direction perpendicular to the chord direction. The invention is defined as in claim 1.

### Advantageous Effects of Invention

According to the dilator of the present invention configured as described above, rapid change in resistance generated when the largest outer diameter portion passes through the introduction hole is suppressed by the cross-sectional area reduction portion, and as a result sensuous resistance sensed by an operator is reduced, and therefore smooth insertion of the dilator can be performed.

If a plurality of the cross-sectional area reduction portions are provided, rapid change in resistance generated when the largest outer diameter portion passes through the introduction hole is more efficiently suppressed, and as a result sensuous resistance sensed by an operator is reduced, and therefore smooth insertion of the dilator can be performed.

If the cross-sectional area reduction portion is provided in at least one of positions facing in the radial direction of the cross section of the largest outer diameter portion, although, when an operator inserts the dilator obliquely with respect to the introduction hole, a portion where the cross-sectional area reduction portion of the largest outer diameter portion is not provided, passes through one of portions in which rapid resistance change easily occurs and angles formed by the dilator and the introduction hole opposing each other in the radial direction are an acute angle and an obtuse angle, since the cross-sectional area reduction portion can pass through the other portion thereof, rapid change in resistance generated when the largest outer diameter portion passes through the introduction hole is easily and reliably suppressed, and accordingly the function of the present invention of reducing the resistance sensed by an operator at the time of insertion is readily realized.

According to the other dilator of the present invention configured as described above, rapid change in resistance generated when the portion in which the rate of increase of the outer diameter of the distal portion of the dilator changes passes through the introduction hole, is suppressed by the cross-sectional area reduction portion, and as a result sensuous resistance sensed by an operator is reduced, and therefore smooth insertion of the dilator can be performed.

### Brief Description of Drawings

[Fig. 1] Fig. 1 is a perspective view of a dilator of a first embodiment.
[Fig. 2] Fig. 2 is a cross-sectional view taken along line II-II of Fig. 1.
[Fig. 3] Fig. 3 is a cross-sectional view taken along line III-III of Fig. 1.
[Fig. 4] Fig. 4 is a schematic configuration diagram showing separated dilator and introducer sheath.
[Fig. 5] Fig. 5 is a schematic configuration diagram showing combination of a dilator and an introducer sheath.
[Fig. 6] Fig. 6 is a graph showing a change in resistance when a diameter of an introduction hole is expanded by a dilator of Comparative Example which does not have a cross-sectional area reduction portion.
[Fig. 7] Fig. 7 is a graph showing a change in resistance when a diameter of an introduction hole is expanded by a dilator created based on a first embodiment.
[Fig. 8] Fig. 8 is a cross-sectional view of a dilator created based on a conventional technology.
[Fig. 9] Fig. 9 is a cross-sectional view taken along line IX-IX of Fig. 8.
[Fig. 10] Fig. 10 is a cross-sectional view of another dilator created based on a conventional technology.
[Fig. 11] Fig. 11 is a cross-sectional view taken along line XI-XI of Fig. 10.
[Fig. 12] Fig. 12 is a graph showing a change in resistance when a diameter of an introduction hole is expanded by a dilator created based on a conventional technology.
[Fig. 13] Fig. 13 is a graph showing a change in resistance when a diameter of an introduction hole is expanded by the other dilator created based on a conventional technology.
[Fig. 14] Fig. 14 is a diagram showing a state in which a dilator is inserted into an introduction hole formed on the skin.
[Fig. 15] Fig. 15 is a diagram showing an enlarged distal portion of a dilator of a second embodiment.
[Fig. 16] Fig. 16 is a cross-sectional view taken along line XVI-XVI of Fig. 15.
[Fig. 17] Fig. 17 is a cross-sectional view taken along line XVII-XVII of Fig. 15.
[Fig. 18] Fig. 18 is a cross-sectional view showing Modification Example of a dilator of an embodiment.
[Fig. 19] Fig. 19 is a cross-sectional view showing the other Modification Example of a dilator of an embodiment.
[Fig. 20] Fig. 20 is a cross-sectional view showing the other Modification Example of a dilator of an embodiment.

### Description of Embodiments

Hereinafter, embodiments of the present invention will be described with reference to the drawings. Dimensional ratios of the drawings are magnified and may be different from actual ratios, for convenience of description.

### <First Embodiment>

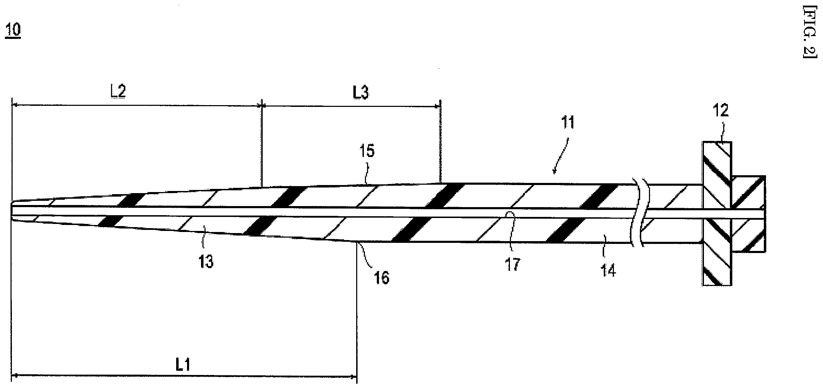

To describe Fig. 1 and Fig. 2, a dilator 10 of the embodiment is used for diameter expansion of an introduction hole communicating with the inside of a living body, and includes an elongated dilator tube 11 having flexibility, and a dilator hub 12 which is fixed to a proximal end of the dilator tube 11. In addition, the dilator 10 includes an inner cavity 17 which penetrates through the dilator tube 11 and the dilator hub 12 in an axial direction.

As a configuration material of the dilator tube 11, a polymer material such as a polyolefin (for example, polyethylene, polypropylene, polybutene, an ethylene-propylene copolymer, an ethylene-vinyl acetate copolymer, an ionomer, or a mixture of two or more kinds), polyolefin elastomer, a crosslinked body of polyolefin, polyvinyl chloride, polyamide, polyamide elastomer, polyester, polyester elastomer, polyurethane, polyurethane elastomer, a fluorine resin, polycarbonate, polystyrene, polyacetal, polyimide, polyetherimide, or a mixture thereof can be used, for example.

The dilator tube 11 includes a distal portion 13, an outer diameter of which increases towards a proximal end in the axial direction, and a cross-sectional area reduction portion 15 which is provided on a largest outer diameter portion 16 of the distal portion 13 and is obtained by reducing a part of an outer periphery of a circular cross section. In addition, the dilator tube 11 includes a shaft portion 14 which elongates from the distal portion 13 to the proximal end side and has an approximately constant diameter along the axial direction. The outer diameter of the dilator tube 11 increases towards the proximal end side in the axial direction in the distal portion 13, and a rate of increase thereof changes in the largest outer diameter portion. In Fig. 1, the outer diameter of the shaft portion 14 is approximately constant and the rate of increase thereof is approximately zero. The cross-sectional area reduction portion 15 is provided over the portions in which the rates of increase of the outer diameter are different from each other.

The distal portion 13 includes a tapered-shaped outer periphery surface. The shaft portion 14 includes an approximately cylindrical outer periphery surface. The largest outer diameter portion 16 is a boundary portion of the distal portion 13 and the shaft portion 14. The cross-sectional area reduction portion 15 has a cross-sectional area which is linear in the axial direction. The cross-sectional area reduction portion 15 has a cross-sectional area which is linear in a circumferential direction as shown in Fig. 3. The cross-sectional area reduction portion 15 is a thin and long elliptical flat surface. The cross-sectional area reduction portion 15 elongates from the largest outer diameter portion 16 to the distal portion 13, and also elongates to the shaft portion 14.

For example, the cross-sectional area reduction portion 15 is formed by chamfering of a part of an edge formed by intersection of the tapered-shaped outer periphery surface of the distal portion 13 and the approximately cylindrical shaped outer periphery surface of the shaft portion 14 from the proximal end of the distal portion 13 to the distal end of the shaft portion 14.

A length L1 of the distal portion 13 in the axial direction is, for example, 20 mm to 25 mm. A length L2 from the distal endmost portion of the dilator tube 11 to the distal end of the cross-sectional area reduction portion 15 in the axial direction is, for example, 8 mm to 15 mm. In addition, a length L3 of the cross-sectional area reduction portion 15 in the axial direction is, for example, 5 mm to 25 mm, and more preferably 8 mm to 15 mm.

As shown in Fig. 1, the cross-sectional area reduction portion 15 elongates to be equally divided between the distal portion 13 and the shaft portion 14, with the largest outer diameter portion 16 as the center.

As shown in Fig. 3, the dilator tube 11 includes three cross-sectional area reduction portions 15. Three cross-sectional area reduction portions 15 are disposed around a shaft center O at regular intervals. A width W1 of the cross-sectional area reduction portion 15 in a chord direction is equal to or greater than a depth D1 of the cross-sectional area reduction portion 15 in a direction perpendicular to the chord direction. The depth D1 is a difference between a length R1 from the shaft center O to a circular arc outer periphery which is different from the cross-sectional area reduction portion 15, and a length R2 of a perpendicular line from the shaft center O to the cross-sectional area reduction portion 15 (D1 = R1 - R2).

The width W1 is, for example, 0.2 mm to 5.0 mm, and more preferably 0.3 mm to 1.0 mm. The depth D1 is, for example, 0.01 mm to 1.0 mm, and more preferably 0.05 mm to 0.3 mm.

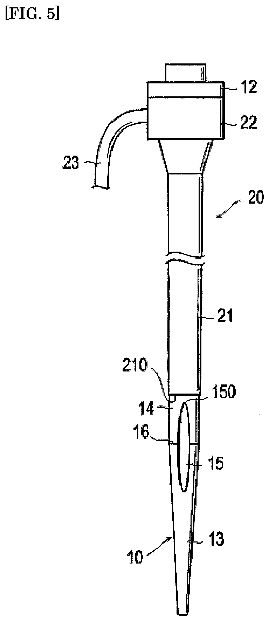

As shown in Fig. 5, a proximal portion 150 of the cross-sectional area reduction portion 15 is positioned at the distal end side with respect to a sheath distal end 210, when the dilator hub 12 of the dilator 10 is fixed with a sheath hub 22 of a sheath introducer sheath 20.

Next, a diameter expansion method of an introduction hole performed by the dilator 10 will be described using insertion of a catheter into a blood vessel by a Seldinger method as an example.

First, an operator punctures a leg or an arm of a patient with a tubular introduction needle (not shown) including a needle tip, to form an introduction hole which communicates with the blood vessel on the skin. Then, the operator inserts a guide wire into the blood vessel through the introduction needle, while maintaining the insertion of the introduction needle into the blood vessel. After the insertion of the guide wire, the operator extracts the introduction needle. After that, the operator inserts the dilator 10 into the introduction hole along the guide wire, to expand the diameter of the introduction hole.

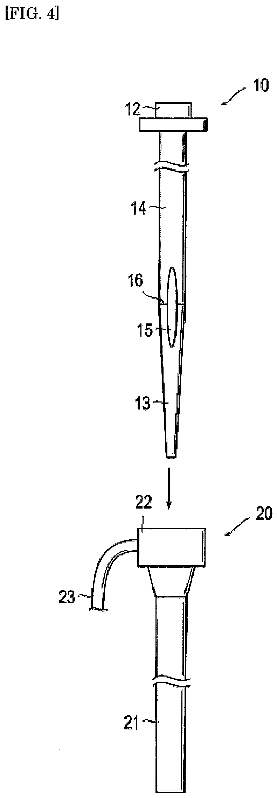

As shown in Fig. 4 and Fig. 5, the operator inserts the introducer sheath 20 and the dilator 10 into the introduction hole, in a state where the dilator 10 is inserted into the tubular introducer sheath 20. At that time, the operator inserts the introducer sheath 20 and the dilator 10 into the introduction hole along the guide wire, by passage of the guide wire through the inner cavity 17.

The introducer sheath 20 includes a flexible sheath tube 21, a sheath hub 22 which is fixed to a proximal end of the sheath tube 21, and a liquid injection tube 23 for liquid injection of saline or the like which communicates with the sheath tube 21 through the sheath hub 22.

The operator inserts the dilator 10 and the introducer sheath 20 into the introduction hole, in a state where the entire cross-sectional area reduction portion 15 is protruded from the distal end of the sheath tube 21 and the dilator 10 and the introducer sheath 20 are fixed by the dilator hub 12 and the sheath hub 22. Since the dilator hub 12 is fixed to the sheath hub 22, the extraction of the dilator 10 from the distal end of the sheath tube 21 is prevented.

The dilator 10 passes through the introduction hole and expands the diameter of the introduction hole by the distal portion 13. Before the cross-sectional area reduction portion 15 reaches the introduction hole, the entire outer periphery of distal portion 13 contacts the introduction hole to expand the diameter of the introduction hole. On the other hand, when the cross-sectional area reduction portion 15 reaches the introduction hole, since the cross-sectional area reduction portion 15 does not easily contact the introduction hole, the distal portion 13 mainly contact the introduction hole in the circular arc outer periphery portion other than the cross-sectional area reduction portion 15 to expand the diameter of the introduction hole.

After the cross-sectional area reduction portion 15 passes through the introduction hole, the distal end of the sheath tube 21 sequentially passes through the introduction hole to be inserted into the blood vessel. After inserting the sheath tube 21 into the blood vessel by a desirable length, the operator extracts the dilator 10 while placing the sheath tube 21 in the blood vessel. The introducer sheath 20 is placed in the living body in a state where the proximal end is extracted to the outside of the body to exhibit a function of communicating the inside and the outside of the living body. The operator introduces a treatment tool such as a guide wire and a catheter into the living body through the introducer sheath 20 placed as described above.

The operation effect of the embodiment will be described.

According to the dilator 10, rapid change in resistance generated when the largest outer diameter portion 16 passes through the introduction hole is suppressed by the cross-sectional area reduction portion 15, and as a result sensuous resistance sensed by an operator is reduced, and therefore smooth insertion of the dilator 10 can be performed.

The inventors practically confirmed such an effect in experiments performed using the dilator created based on the embodiment. The experiments will be simply described hereinafter.

The inventors created a dilator having a configuration the same as the embodiment, and a dilator which did not include a cross-sectional area reduction portion, that is, which included a circular arc edge over the entire periphery of the largest outer diameter portion, as a comparison target. In both dilators, the main configurations other than the cross-sectional area reduction portion were the same as each other. The width W1 of the cross-sectional area reduction portion was 1.050 mm, and the depth D1 of the cross-sectional area reduction portion was 0.163 mm (average value of depth D1 of three cross-sectional area reduction portions). The inventors obliquely inserted the dilators into an introduction hole formed on a film obtained by imitating the skin, and measured changes in force applied to the dilators at that time.

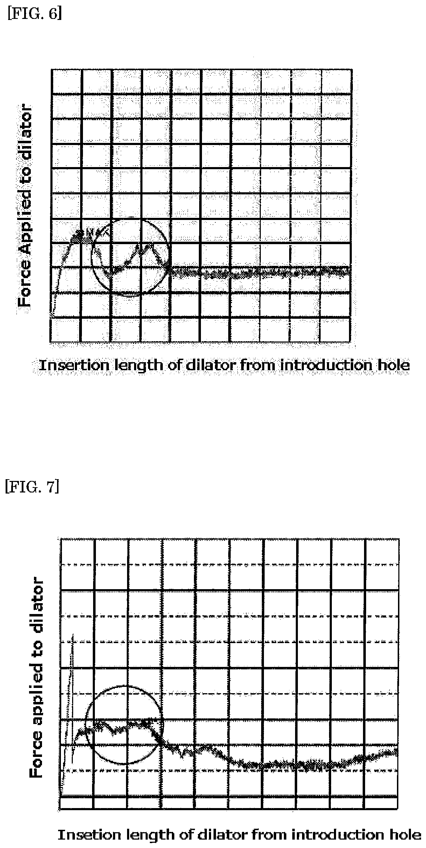

As shown in Fig. 6, in the experiment using the dilator not including the cross-sectional area reduction portion, rapid increase in resistance was confirmed, when the largest outer diameter portion passes through the introduction hole (see portion surrounded by a circle). On the other hand, as shown in Fig. 7, in the experiment using the dilator including the cross-sectional area reduction portion, rapid increase in resistance described above was not confirmed (see portion surrounded by a circle). From this result, it was found that the rapid change in resistance generated when the largest outer diameter portion passes through the introduction hole was suppressed by the cross-sectional area reduction portion. In addition, in the case of using the dilator including the cross-sectional area reduction portion, sensuous resistance when the largest outer diameter portion passes through the introduction hole was also smaller than in the case without the cross-sectional area reduction portion. In the experimental result shown in Fig. 7, a peak appearing immediately after the starting of the insertion is caused by a burr remaining on the distal end of the dilator, and is not caused by the cross-sectional area reduction portion.

For comparison with the embodiment, the inventors created two kinds of dilators 30 and 40 based on the conventional technology disclosed in above Citation List, and practically confirmed the effects thereof by the experiment.

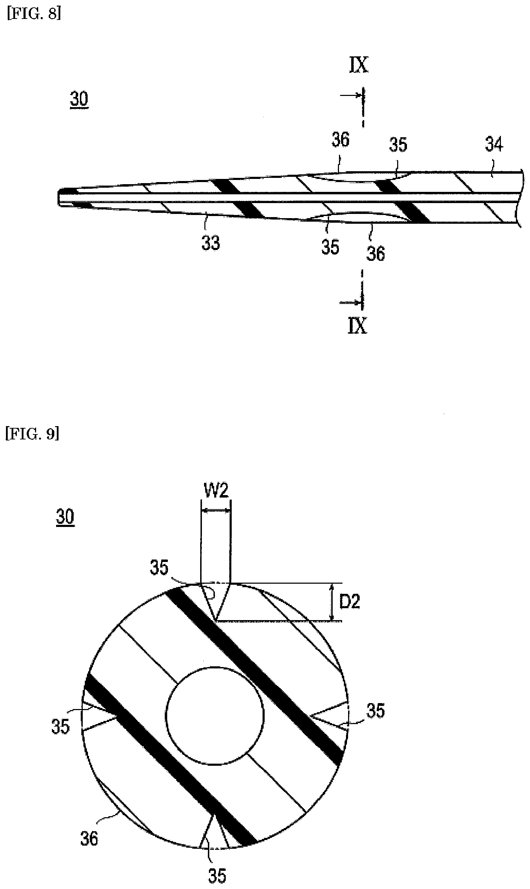

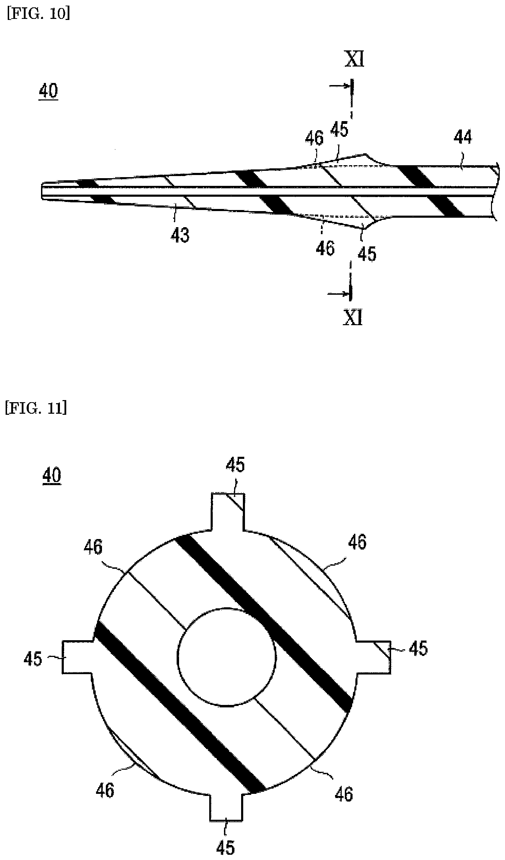

As shown in Fig. 8 and Fig. 9, the dilator 30 includes a slit-shaped groove 35 which passes through a largest outer diameter portion 36 and elongates from a distal portion 33 to a shaft portion 34. A width W2 of the groove 35 is smaller than a depth D2 of the groove 35 (W2 < D2). The width W2 of the groove 35 is 0.262 mm, and the depth D2 of the groove 35 is 0.315 mm (average value of depth D2 of four grooves 35). As shown in Fig. 10 and Fig. 11, the dilator 40 includes a protrusion 45 which passes through a largest outer diameter portion 46 and elongates from a distal portion 43 to a shaft portion 44. The inventors obliquely inserted the dilators 30 and 40 into the introduction hole formed on a film obtained by imitating the skin, and measured changes in force applied to the dilators 30 and 40 at that time.

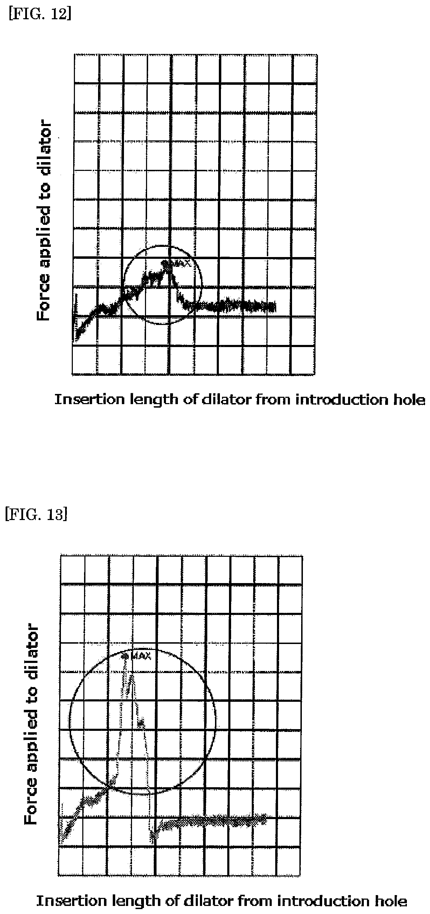

As shown in Fig. 12, in a case of using the dilator 30, there is a sharp change in the graph before and after the largest outer diameter portion 36 passes through the introduction hole, compared to the case of the embodiment shown in Fig. 6 (see portion surrounded by a circle). That is, the resistance changes largely. In addition, the sensuous resistance sensed when the largest outer diameter portion 36 passes through the introduction hole is also large.

As shown in Fig. 13, in a case of using the dilator 40, there is a sharp change in the graph before and after the largest outer diameter portion 46 passes through the introduction hole, compared to the case of the embodiment shown in Fig. 6 (see portion surrounded by a circle). That is, the resistance changes largely. In addition, the sensuous resistance sensed when the largest outer diameter portion 46 passes through the introduction hole is also large.

From this result, it was confirmed that, with the slit-shaped groove 35 or the protrusion 45 having greater depth than the width as in the related art, the rapid change in resistance generated when the largest outer diameter portion passes through the introduction hole cannot be suppressed unlike the embodiment, and the sensuous resistance sensed by the operator is also greater compared to the case of the embodiment.

The dilator 10 of the embodiment includes the plurality of cross-sectional area reduction portions 15, and accordingly, compared to the case of including one cross-sectional area reduction portion 15, the rapid change in resistance generated when the largest outer diameter portion 16 passes through the introduction hole is more efficiently suppressed, and as a result the sensuous resistance sensed by the operator is further reduced, and therefore smoother insertion of the dilator 10 can be performed.

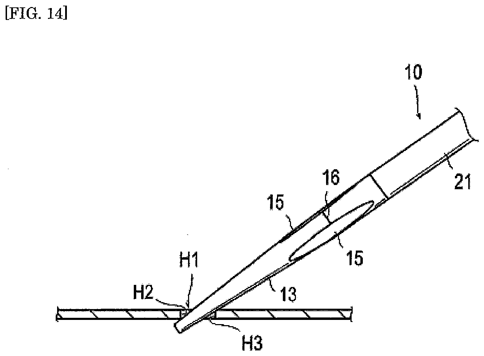

In addition, the cross-sectional area reduction portion 15 is provided in one of positions facing in the radial direction of the cross section of the largest outer diameter portion 16. Accordingly, when the operator inserts the dilator 10 obliquely with respect to an introduction hole H1 as shown in Fig. 14, although a portion where the cross-sectional area reduction portion 15 in the largest outer diameter portion 16 is not provided (portion opposing the cross-sectional area reduction portion 15 in the radial direction, to describe with Fig. 3), passes through one of portions H3 and H2 in which rapid resistance change easily occurs and angles formed by the dilator 10 and the introduction hole H1 opposing each other in the radial direction are an acute angle and an obtuse angle, the cross-sectional area reduction portion 15 can pass through the other portion thereof. Accordingly, the rapid change in resistance sensed when the largest outer diameter portion 16 passes through the introduction hole H1 is easily and reliably suppressed, and therefore the function of the dilator 10 of reducing the resistance sensed by the operator at the time of insertion is readily realized.

Since the cross-sectional area reduction portion 15 has a cross-sectional area which is linear in the axial direction, change in angles between the introduction hole and the distal portion 13 or the shaft portion 14 of the dilator 10 is moderated when the distal portion or the proximal portion of the cross-sectional area reduction portion 15 passes through the introduction hole, and accordingly the resistance sensed by the operator can be reduced.

Since the cross-sectional area reduction portion 15 has a cross-sectional area which is linear in the circumferential direction, change in angles between the introduction hole and the distal portion 13 or the shaft portion 14 is moderated in a case where the operator inserts the dilator 10 while twisting it, when the cross-sectional area reduction portion 15 passes through the introduction hole, and accordingly the resistance sensed by the operator can be reduced.

Since the cross-sectional area reduction portion 15 is a thin and long elliptical flat surface, change in resistance in the axial direction or the circumferential direction generated when the distal portion 13 of the dilator 10 passes through the introduction hole is suppressed.

As shown in the drawing, since the cross-sectional area reduction portion 15 elongates to be equally divided between the distal portion 13 and the shaft portion 14, with the largest outer diameter portion 16 as the center, change in resistance generated when the distal portion and the proximal portion of the cross-sectional area reduction portion 15 which is provided on the distal portion 13 of the dilator 10 pass through the introduction hole, respectively, can be moderated.

### <Second Embodiment>

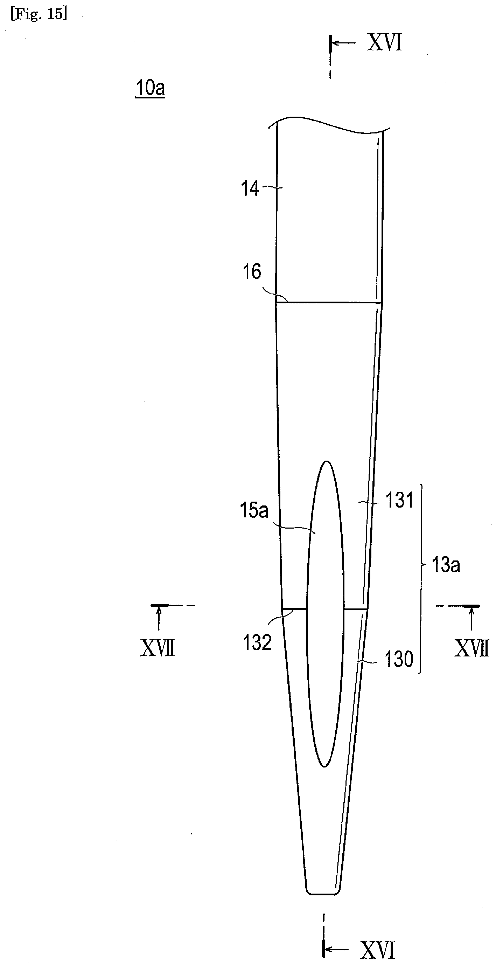

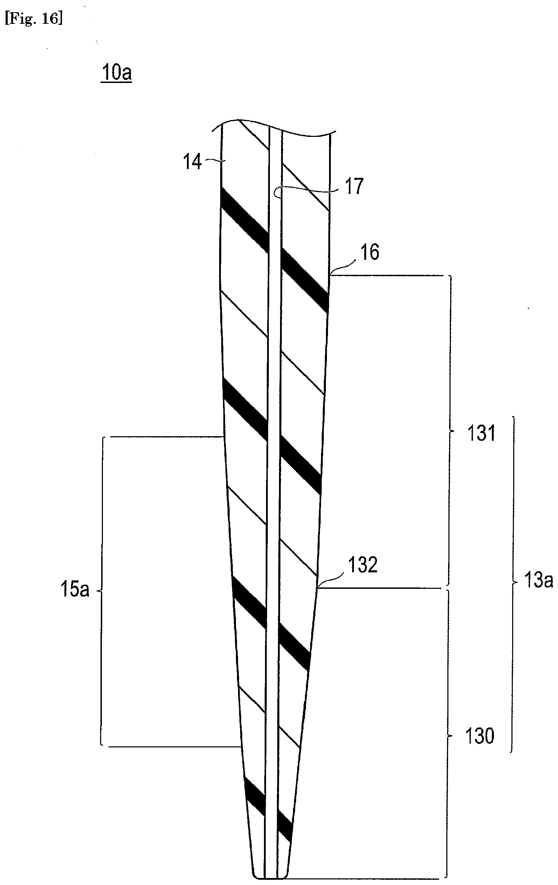

As shown in Figs. 15 to 17, in a dilator 10a of a second embodiment, a shape of a distal portion 13a and a position of a cross-sectional area reduction portion 15a are different from those of the first embodiment. Since the other configurations and the method of use of the second embodiment are substantially the same as those of the first embodiment, overlapping descriptions will be omitted.

While the rate of increase of the outer diameter is constant in the distal portion 13 of the first embodiment, a rate of increase of an outer diameter changes in the distal portion 13a of the second embodiment. The distal portion 13a includes a first section 130 and a second section 131 which have a different rate of increase of an outer diameter from each other. The rate of increase of the outer diameter of the first section 130 provided on a distal end side is larger than the rate of increase of the outer diameter of the second section 131 provided on a proximal end side.

The cross-sectional area reduction portion 15a is provided on a boundary portion 132 of the first section 130 and the second section 131, that is, a portion in which the rate of increase of the outer diameter changes in the distal portion 13a, not on the largest outer diameter portion 16. In addition, the cross-sectional area reduction portion 15a elongates to a distal end side and a proximal end side of the boundary portion 132. The cross-sectional area reduction portion 15a is provided on a position different from the cross-sectional area reduction portion 15 of the first embodiment, however the configuration of the cross-sectional area reduction portion 15a itself is substantially the same as the cross-sectional area reduction portion 15.

According to the dilator 10a, rapid change generated when the boundary portion 132 passes through the introduction hole is suppressed by the cross-sectional area reduction portion 15a, and as a result sensuous resistance sensed by the operator is reduced, and therefore smooth insertion of the dilator 10a can be performed.

The dilator 10a includes the plurality of cross-sectional area reduction portions 15a, and accordingly, compared to the case of including one cross-sectional area reduction portion 15a, the rapid change in resistance generated when the boundary portion 132 passes through the introduction hole is more efficiently suppressed, and as a result the sensuous resistance sensed by the operator is further reduced, and therefore smoother insertion of the dilator 10a can be performed.

In addition, the cross-sectional area reduction portion 15a is provided in one of positions facing in the radial direction of the cross section of the boundary portion 132. Accordingly, even when the portion in which the cross-sectional area reduction portion 15a is not provided passes through any one of the portions H2 and H3 of the introduction hole in which the rapid change in resistance easily occurs, the cross-sectional area reduction portion 15a can pass through the other portion thereof. Accordingly, the rapid change in resistance generated when the boundary portion 132 passes through the introduction hole is easily and reliably suppressed, and therefore the function of the dilator 10a of reducing the resistance sensed by the operator at the time of insertion is readily realized.

Since the cross-sectional area reduction portion 15a has a cross-sectional area which is linear in the axial direction, change in angles between the introduction hole and the first section 130 or the second section 131 of the dilator 10a is moderated when the distal portion or the proximal portion of the cross-sectional area reduction portion 15a passes through the introduction hole, and accordingly the resistance sensed by the operator can be reduced.

Since the cross-sectional area reduction portion 15a has a cross-sectional area which is linear in the circumferential direction, change in angles between the introduction hole and the outer periphery of the first section 130 or the second section 131 is moderated in a case where the operator inserts the dilator 10 while twisting it, when the cross-sectional area reduction portion 15a passes through the introduction hole, and accordingly the resistance sensed by the operator can be reduced.

Since the cross-sectional area reduction portion 15a is a thin and long elliptical flat surface, change in resistance in the axial direction or the circumferential direction generated when the distal portion 13a of the dilator 10a passes through the introduction hole is suppressed.

As shown in the drawing, since the cross-sectional area reduction portion 15a elongates to be equally divided between the first section 130 and the second section 131, with the boundary portion 132 as the center, change in resistance generated when the distal portion and the proximal portion of the cross-sectional area reduction portion 15a which is provided on the distal portion 13a of the dilator 10a pass through the introduction hole, respectively, can be moderated.

The present invention is not limited to the embodiments described above, and various modifications can be performed within a scope of the claims.

For example, the number of the cross-sectional area reduction portions is not limited to three, and may be four or more. The number of the cross-sectional area reduction portions can be suitably set based on viewpoints of improvement of intensity of the dilator and suppression of the change in resistance at the time of inserting into the introduction hole. The plurality of cross-sectional area reduction portions may not be disposed symmetrically around the shaft center.

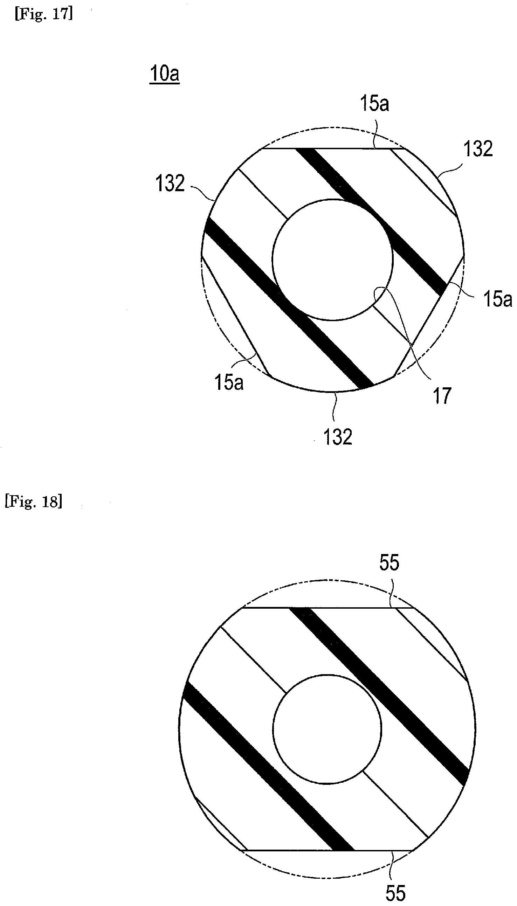

As shown in Fig. 18, the cross-sectional area reduction portions may be provided in both positions facing in the radial direction in the cross section of the largest outer diameter portion or in the cross section of the portion in which the rate of increase of the outer diameter changes in the distal portion. By providing the cross-sectional area reduction portions as described above, the cross-sectional area reduction portions can pass through both the portions H2 and H3 of the introduction hole described above in which the rapid change in resistance easily occurs. Accordingly, the rapid change in resistance generated when the largest outer diameter portion passes through the introduction hole is more efficiently suppressed.

In addition, the cross-sectional area reduction portion is not limited to the flat surface of the embodiment, and may be a recessed curved surface 65, as shown in Fig. 19. In this case, a depth D3 of the cross-sectional area reduction portion is a distance from a chord connecting the cut circular arc (dashed-two dotted line in the drawing) to the deepest portion of the curved surface 65. In addition, the recessed shape is not limited to the circular arc as shown in the drawing, and may be a rectangular shape.

As shown in Fig. 20, the dilator may include reinforcing bodies 79 which are provided in cross-sectional area reduction portions 75. The reinforcing body 79 is, for example, a protrusion which elongates in the axial direction of the dilator. The rapid change in resistance when increasing the intensity of the dilator and inserting the dilator into the introduction hole can be suppressed by the reinforcing body 79.

Priority is claimed on Japanese Patent Application No. 2011-168776, filed August 1, 2011.

### Reference Signs List

10 Dilator of first embodiment
10a Dilator of second embodiment
11 Dilator tube
12 Dilator hub
13 Distal portion
13a Distal portion
14 Shaft portion
15 Cross-sectional area reduction portion
15a Cross-sectional area reduction portion
16 Largest outer diameter portion
17 Inner cavity
20 Introducer sheath
21 Sheath tube
22 Sheath hub
30, 40 Dilator of Comparative Example
35 Slit-shaped groove
36 Largest outer diameter portion
45 Protrusion
46 Largest outer diameter portion
55, 65, 75 Cross-sectional area reduction portion
79 Reinforcing body
130 First section
131 Second section
132 Boundary portion (portion in which rate of increase of outer diameter changes)
H1 Introduction hole
W1, W3 Width of cross-sectional area reduction portion
D1, D3 Depth of cross-sectional area reduction portion

## Claims

1. A dilator (10) comprising:
a distal portion (13), an outer diameter of which increases towards a proximal end side in an axial direction defining a tapered-shaped outer surface;
a shaft portion (14) which elongates from the distal portion (13) to the proximal end side; and
a cross-sectional area reduction portion (15) which is provided on a largest outer diameter portion (16) of the distal portion (13), and is obtained by reducing a part of an outer periphery of a circular cross section;
wherein a width of the cross-sectional area reduction portion (15) of the largest outer diameter portion in a chord direction is equal to or greater than a depth of the cross-sectional area reduction portion of the largest outer diameter portion (16) in a direction perpendicular to the chord direction;
**characterized in that**
a plurality of the cross-sectional area reduction portions are provided;
the largest outer diameter portion (16) is a boundary portion of the distal portion and the shaft portion (14); and
the cross-sectional area reduction portion (15) further elongates from the largest outer diameter portion (16) of the distal portion (13), to the shaft portion (14).

2. The dilator according to claim 1, wherein the cross-sectional area reduction portion (15) is provided in at least one of positions facing in a radial direction of the cross section of the largest outer diameter portion.

3. The dilator according to claim 1, wherein the cross-sectional area reduction portion (15) is formed by chamfering of a part of an edge formed by intersection of the tapered-shaped outer periphery surface of the distal portion (13) and the approximately cylindrical shaped outer periphery surface of the shaft portion (14) from the proximal end of the distal portion (13) to the distal end of the shaft portion.

## Patentansprüche

1. Dilatator (10), umfassend:
einen distalen Abschnitt (13), dessen Außendurchmesser in einer axialen Richtung in Richtung einer Seite eines proximalen Endes steigt, wodurch eine kegelförmige Außenfläche definiert wird;
einen Schaftabschnitt (14), welcher sich von dem distalen Abschnitt (13) zu der Seite des proximalen Endes erstreckt; und
einen Querschnittsflächen-Reduktionsabschnitt (15), welcher an einem Abschnitt größten Außendurchmessers (16) des distalen Abschnitts (13) vorgesehen ist und durch Verringern eines Teils eines Außenrandes eines kreisförmigen Querschnitts erhalten wird;
wobei eine Breite des Querschnittsflächen-Reduktionsabschnitts (15) des Abschnitts größten Außendurchmessers in einer Sehnenrichtung größer oder gleich einer Tiefe des Querschnittsflächen-Reduktionsabschnitts des Abschnitts größten Außendurchmessers (16) in einer Richtung senkrecht zu der Sehnenrichtung ist;
**dadurch gekennzeichnet, dass**
mehrere der Querschnittsflächen-Reduktionsabschnitte vorgesehen sind;
der Abschnitt größten Außendurchmessers (16) ein Grenzabschnitt des distalen Abschnitts und des Schaftabschnitts (14) ist und
sich der Querschnittsflächen-Reduktionsabschnitt (15) ferner von dem Abschnitt größten Außendurchmessers (16) des distalen Abschnitts (13) zu dem Schaftabschnitt (14) erstreckt.

2. Dilatator nach Anspruch 1, wobei der Querschnittsflächen-Reduktionsabschnitt (15) in mindestens einer von Positionen vorgesehen ist, welche in eine radiale Richtung des Querschnitts des Abschnitts größten Außendurchmessers zeigen.

3. Dilatator nach Anspruch 1, wobei der Querschnittsflächen-Reduktionsabschnitt (15) durch Abschrägen eines Teils eines Randes, der durch Schnitt der kegelförmigen äußeren Randfläche des distalen Abschnitts (13) mit der ungefähr zylindrisch geformten äußeren Randfläche des Schaftabschnitts (14) gebildet wird, von dem proximalen Ende des distalen Abschnitts (13) zu dem distalen Ende des Schaftabschnitts gebildet wird.

## Revendications

1. Dilatateur (10) comprenant :
une partie distale (13) dont un diamètre extérieur augmente vers un côté d'extrémité proximale dans une direction axiale définissant une surface extérieure de forme effilée ;
une partie d'arbre (14) qui s'allonge depuis la partie distale (13) jusqu'au côté d'extrémité proximale ; et
une partie de réduction de superficie de section transversale (15) qui est fournie au niveau d'une partie à diamètre extérieur le plus grand (16) de la partie distale (13), et est obtenue en réduisant une partie d'une périphérie extérieure d'une section transversale circulaire ;
dans lequel une largeur de la partie de réduction de superficie de section transversale (15) de la partie à diamètre extérieur le plus grand dans une direction de corde est supérieure ou égale à une profondeur de la partie de réduction de superficie de section transversale de la partie à diamètre extérieur le plus grand (16) dans une direction perpendiculaire à la direction de corde ;
**caractérisé en ce**
**qu'**une pluralité des parties de réduction de superficie de section transversale sont fournies ;
**que** la partie à diamètre extérieur le plus grand (16) est une partie limitrophe de la partie distale et de la partie d'arbre (14) ; et
**que** la partie de réduction de superficie de section transversale (15) s'allonge en outre depuis la partie à diamètre extérieur le plus grand (16) de la partie distale (13) jusqu'à la partie d'arbre (14).

2. Dilatateur selon la revendication 1, dans lequel la partie de réduction de superficie de section transversale (15) est fournie dans au moins l'une des positions faisant face dans une direction radiale de la section transversale de la partie à diamètre extérieur le plus grand.

3. Dilatateur selon la revendication 1, dans lequel la partie de réduction de superficie de section transversale (15) est formée par chanfreinage d'une partie d'un bord formé par l'intersection entre la surface périphérique extérieure de forme effilée de la partie distale (13) et la surface périphérique extérieure de forme approximativement cylindrique de la partie d'arbre (14) depuis l'extrémité proximale de la partie distale (13) jusqu'à l'extrémité distale de la partie d'arbre.
